# EUROPEAN PATENT APPLICATION

(11) **EP 3 042 887 A1**
(43) Date of publication of application: **13.07.2016**
(21) Application number: 15305021.6
(22) Date of filing: 12.01.2015
(51) Int. Cl.: C07C 2/40, C07C 5/03

(54) **Catalytic process for diene dimerization**

(71) Applicant: TOTAL MARKETING SERVICES, 92800 Puteaux (FR); CENTRE NATIONAL DE LA RECHERCHE SCIENTIFIQUE -CNRS-, 75794 Paris Cedex 16 (FR)
(72) Inventor: Taoufik, M. Mostafa, 69100 Villeurbanne (FR); Szeto, Kai Chung, 69100 Villeurbanne (FR); Rios Neyra, Cesar, 69100 Villeurbanne (FR)
(74) Representative: Hirsch & Associés

(57) **Abstract**

The invention relates to the dimerization of conjugated diene compounds by a catalytic process in a solvent medium comprising hydrocarbons, in the presence of a specific additive of the phenol type.

## Description

### FIELD OF THE INVENTION

The invention relates to the dimerization of conjugated diene compounds, in particular terminal conjugated diene compounds, by a catalytic process in a solvent medium comprising hydrocarbons, in the presence of a specific additive.

### BACKGROUND OF THE INVENTION

Products obtained by dimerization of conjugated dienes and further hydrogenation may be used in different fields, such as flavors and fragrances, pharmaceutical, cosmetics, solvents and lubricants applications. In cosmetic applications, hydrogenated dimers obtained from conjugated dienes may be used in creams, such as nutrient creams and medicated creams or in toilet or milky lotion, in lipstick or in face powder. In pharmaceutical applications, hydrogenated dimers obtained from conjugated dienes may be used in medical and pharmaceutical preparations such as ointments, and medical lubricating agents. As an example of a useful hydrogenated dimer, special mention can be made to squalane, isosqualane and crocetane, obtained respectively by hydrogenation of squalene, isosqualene and crocetene.

The dimerization process of conjugated dienes is generally performed using a catalyst in the presence of a solvent.

Document US 8,669,403 describes the dimerization of farnesene using 2-propanol as solvent. Document WO 2011/146837 describes processes for catalytic dimerization of farnesene, said processes can be carried out in a protic solvent, such as a primary or a secondary alcohol, such as isopropanol.

The protic solvents are very expensive and are thus not convenient for an industrial process.

There still exists a need for the dimerization of conjugated dienes by an industrial process which would be economical and would present an improved yield and/or selectivity for the desired dimers.

### SUMMARY OF THE INVENTION

A first object of the present invention is a process for the dimerization of conjugated diene compounds comprising contacting, in a solvent medium comprising hydrocarbons, said conjugated diene compounds with a catalyst in the presence of a phenol compound.

According to an embodiment of the invention, the conjugated diene compounds are terminal conjugated diene compounds.

According to an embodiment of the invention, the terminal conjugated diene compounds have the following formula: 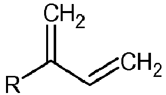 wherein R is a hydrocarbon radical having 1 to 15 carbon atom, optionally comprising one or more heteroatoms, such as nitrogen, oxygen or sulphur.

According to an embodiment, the catalyst is a homogeneous catalyst.

According to another embodiment, the catalyst is a heterogeneous catalyst.

According to an embodiment of the invention, the solvent medium comprises at least 50% by weight of hydrocarbons, preferably at least 70% by weight, more preferably at least 90% by weight of hydrocarbons, still more preferably at least 99% by weight of hydrocarbons.

According to an embodiment, the hydrocarbons comprised in the solvent are chosen from pentane, heptane, hexane, cyclohexane, toluene and xylene.

According to an embodiment of the invention, the phenol compound is phenol.

According to an embodiment, the process of the invention further comprises a hydrogenation step, whereby hydrogenated dimers are obtained.

An advantage of the present invention is a process that involves less expensive solvents than the usual protic solvents. Another advantage of the present invention is a process leading to a satisfying yield and/or selectivity in the desired product.

Further features and advantages of the invention will appear from the following description of embodiments of the invention, given as non-limiting examples, with reference to the accompanying drawings listed hereunder.

### BRIEF DESCRIPTION OF THE FIGURE

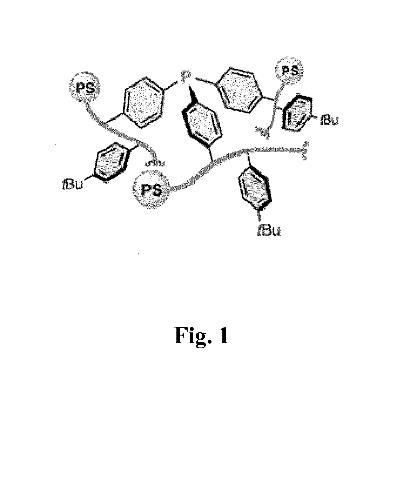

Fig. 1 represents a polystyrene-triphenylphosphane hybrid polymer.

### DETAILED DESCRIPTION OF THE INVENTION

A first object of the present invention is a process for the dimerization of conjugated diene compounds comprising contacting, in a solvent medium comprising hydrocarbons, said conjugated diene compounds with a catalyst in the presence of a phenol compound.

### Diene compound

By "conjugated diene compounds" according to the present invention, it is to be understood a hydrocarbon radical, linear, branched or cyclic, comprising at least two conjugated carbon-carbon double bonds. The hydrocarbon radical may also comprise at least one heteroatom, such as oxygen, nitrogen or sulfur. Preferably, the hydrocarbon radical consists in hydrogen and carbon atoms. The hydrocarbon radical preferably comprises from 4 to 30 carbon atoms, more preferably from 5 to 20 carbon atoms.

According to an embodiment, the conjugated diene compounds are terminal conjugated diene compounds.

According to an embodiment, the terminal conjugated diene compounds have the following formula: 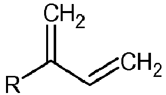 wherein R is a hydrocarbon radical, linear, branched or cyclic, saturated or unsaturated having 1 to 15 carbon atom, optionally comprising one or more heteroatoms, such as nitrogen, oxygen or sulphur.

According to an embodiment, the conjugated diene compounds are chosen from terpenes, such as myrcene or beta-farnesene, beta-phellandrene or alpha-terpinene.

Myrcene refers to a compound having the following formula:

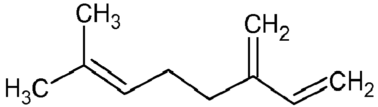

Beta-farnesene refers to a compound having the following formula:

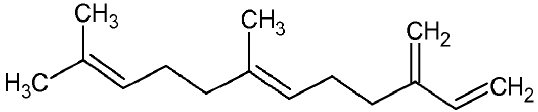

Terpenes are molecules of natural origin, produced by numerous plants, in particular conifers.

By definition, terpenes (also known as isoprenoids) are a class of hydrocarbons bearing as the base unit an isoprene moiety (i.e. 2-methyl-buta-1,3-diene). Isoprene [CH₂=C(CH₃)CH=CH₂] is represented below:

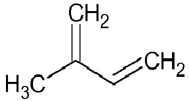

Terpenes may be classified according to the number n (integer) of isoprene units of which it is composed, for example:
n = 2: monoterpenes (C₁₀), such as myrcene;
n = 3: sesquiterpenes (C₁₅), such as farnesene;
n = 4: diterpenes (C₂₀).

Alpha-terpinene is a cyclic terpene having two conjugated carbon-carbon double bonds and refers to a compound having the following formula:

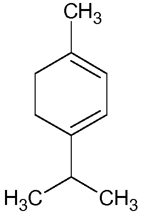

According to an embodiment, the dimerization reaction is performed with conjugated dienes of same chemical nature. According to another embodiment, the dimerization reaction is performed with conjugated dienes of different chemical natures. Preferably, the dimerization reaction is performed with conjugated dienes of same chemical nature.

### Solvent medium

The dimerization process according to the invention comprises the reaction between at least two conjugated diene compounds in a solvent medium comprising hydrocarbons. These hydrocarbons are non protic compounds. They are solvents for the diene compounds. According to the invention, the selected hydrocarbon for the solvent medium is different from the diene compounds described above.

Preferably, the solvent medium comprises at least 50% by weight of hydrocarbons, preferably at least 70% by weight, more preferably at least 90% by weight of hydrocarbons, still more preferably at least 99% by weight of hydrocarbons, based on the total weight of the solvent medium.

The hydrocarbons comprised in the solvent medium may be chosen from a linear, a branched or a cyclic hydrocarbon.

For example, the hydrocarbons may be chosen from pentane, heptane, hexane, cyclohexane, toluene and xylene.

### Catalyst

The catalyst used in the present invention can be a homogeneous catalyst (soluble in the solvent medium) or a heterogeneous catalyst (insoluble in the solvent medium). Catalysts suitable to conduct a dimerization process are known to the one skill in the art.

For example, documents US 8,669,403 and WO 2011/146837 describe some catalysts that can be used in the process of the present invention.

The catalyst may be selected from palladium catalyst, nickel catalyst or zirconium catalyst.

According to one embodiment, the catalyst used is a palladium catalyst. The palladium catalyst may be formed from a palladium precursor selected from [Pd(allyl)Cl]₂, Pd(cod)Cl₂, Pd2(dba)₃, Pd(dba)₂, Pd(dba), Pd(acac)₂, or an equimolar mixture of Pd(dba)₃ and Pd₂(dba)₃. The catalyst may further comprise a ligand selected from triphenyl phosphine, triethyl phosphine and tritolyl phosphine.

### Phenol compound

The process of dimerization according to the present invention is performed in the presence of a phenol compound.

By "phenol compound" according to the present invention, it is to be understood a compound comprising at least one group of formula:

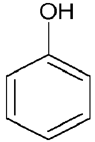

The phenol compound may be a phenol substituted by one or more substituents. The substituents may be chosen from methyl, ethyl or propyl groups.

The inventors surprisingly found that the addition of a phenol compound in the reaction medium improved the yield and/or the selectivity of the dimerization reaction in case a conventional and less expensive solvent is used, such as an hydrocarbon.

Preferably, the phenol compound is a phenol, i.e. a non-substituted phenol.

According to an embodiment, the phenol compound represents, by weight, from 0,2 to 2%, preferably from 0,4 to 1%, ideally around 0,6%, of the reaction medium (solvent + diene + phenol).

### Reaction process

The reaction is preferably performed at a temperature comprised between 50°C and 150°C, preferably between 25°C and 95°C. At higher temperatures, there is a risk that the diene polymerizes.

The reaction is preferably performed in an inert gas atmosphere, for example in argon or nitrogen atmosphere, preferably at atmospheric pressure.

The reaction is preferably performed during at least 5 hours, preferably at least 8 hours, more preferably during from 8 to 36 hours, ideally from 12 to 24 hours.

The reaction is preferably performed with a molar ratio conjugated dienes/catalyst ranging from 200 to 5000, preferably from 500 to 3000, more preferably from 1000 to 2000.

The reaction is preferably performed with a molar ratio phenol compound/catalyst ranging from 10 to 200, preferably from 20 to 100.

The reaction can be a batch reaction, a semi-batch reaction or a continuous reaction and preferably takes place in a stirred reactor. Upon completion of the reaction, which under the said process conditions yields a high selectivity and a high conversion, the resulting dimerization product can be separated off from the reactor stream in a manner known per se, for instance by distillation, absorption, etc.

The dimerization product can further be submitted to a hydrogenation reaction in the presence of a hydrogenation catalyst. The step of hydrogenation may be carried out by methods well known for the skilled person. For example, the step of hydrogenation may be performed with hydrogen in the presence of a hydrogenation catalyst, such as Pd/C, Raney nickel or Ni/Al₂O₃.

After hydrogenation, hydrogenated dimers are obtained, such as crocetane, squalane or isosqualane, hydrogenated dimer of alpha-terpinene, hydrogenated dimer of beta-phellandrene.

Preferably, dimers obtained after the hydrogenation are saturated dimers.

The process according to the present invention allows improving the selectivity of the process. In particular, the use of the combination of the hydrocarbon-containing solvent medium and the phenol compound allows improving the selectivity for the head-to-head dimer.

The "selectivity for compound X" refers to the amount of compound X formed in the dimerization reaction based on the total amount of products formed. The selectivity is expressed as a percentage by weight.

### EXAMPLES

Examples have been performed using myrcene as conjugated diene, and a Pd based heterogeneous catalyst (polystyrene-triphenylphosphane hybrid polymer, such as described in Fig. 1):
- Example 1: reaction with heptane as solvent and phenol as additive
- Example 2: reaction with isopropanol as solvent and phenol as additive
- Example 3: reaction with isopropanol as solvent without phenol.

For examples 1 and 2, the protocol was as follows:

The phosphine hybrid polymer (0.200 g, 0.018 mmol, 1 eq), Palladium(II) acetylacetonate catalyst (5.5 mg, 0.018 mmol, 1 eq.), Myrcene (molar ratio myrcene/catalyst = 2000, 4.9 g, 36 mmol) and Phenol (0.101 g, 1.08 mmol, 60 eq.) were charged in a 100 mL schlenk. Then, 15 mL of solvent (isopropanol or heptane) was added under atmospheric pressure of Argon or nitrogen to this mixture and stirred for 12 h at 100°C. Finally, the crude of the dimerization reaction was filtrated through a silica path on Büchner fritted disc funnel and washed several times with toluene. The solvent was evaporated in the rotavapor. The crude (0.160 g) was then hydrogenated in an auto-clave (100 mL) with 7 mL of Toluene, 0.150 g of Pd/C (10 wt% load), 40 bar of hydrogen at 85°C for 12 h. Finally, an aliquot of the product with an internal standard dodecane was injected in GC-FID. The results are presented in the table 1.

For example 3, the protocol was as follows:

The phosphine hybrid polymer (0.200 g, 0.018 mmol, 1 eq), Palladium(II) acetylacetonate catalyst (5.5 mg, 0.018 mmol, 1 eq), Myrcene (molar ratio myrcene/catalyst = 500, 1.2 g, 9 mmol) were charged in a 100 mL schlenk. Then, 15 mL of isopropanol was added under atmospheric pressure of Argon or nitrogen to this mixture and stirred for 12 h at 100°C. Finally, the crude of the dimerization reaction was filtrated through a silica path on Büchner fritted disc funnel and washed several times with toluene. The solvent was evaporated in the rotavapor. The crude (0.160 g) was then hydrogenated in an auto-clave (100 mL) with 7 mL of Toluene, 0.150 g of Pd/C (10 wt% load), 40 bar of hydrogen at 85°C for 12 h. Finally, an aliquot of the product with an internal standard dodecane was injected in GC-FID. The results are presented in the table 2.

### Conditions for the hydrogenation - for the analysis of the dimerization products

The crude of the dimerization reaction (0.089 g) was charged in a stainless steel autoclave with 10 wt% Pd/C (150 mg), 5 mL of toluene, 40 bar of H₂ and stirred for 12 h at 85 °C. After that, an internal standard nonadecane (80 mg) was added to the hydrogenated mixture and an aliquot was injected in the GC-FID to obtain the conversion and selectivity on dimers. The conversion was mainly calculated based on the latter method unless further specification.

The chemical reactions involved in the examples are the following:

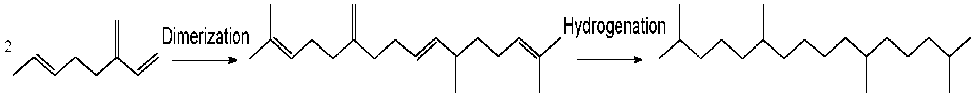

The conversion and the selectivity obtained in the examples are indicated in the two tables below.

**Table 1: conversion and selectivity of examples 1 and 2**

| | Myrcene conversion (%) | Selectivity (%) | | | | | |
|---|---|---|---|---|---|---|---|
| | | Head-to-head dimer | Other dimers | trimers | Phenoxydimer | Head-to-tail dimer | Molar ratio (head-to-head dimer/head-to-tail dimer) |
| Ex. 1 | 68 | 77.7 | 4.9 | 4.1 | 4.3 | 9.0 | 8.6 |
| Ex. 2 | 90 | 60.8 | 18.9 | 15.8 | - | 4.5 | 13.4 |

**Table 2: conversion and selectivity of example 3**

| Example 3 | Myrcene conversion (%) | Selectivity (%) | | | | |
|---|---|---|---|---|---|---|
| | | Head-to-head dimer (crocetane) | Other dimers | trimers | Head-to-tail dimer | Molar ratio (head-to-head dimer/head-to-tail dimer) |
| | 88 | 65,5 | 9,1 | 21,7 | 3,7 | 17,7 |

Example 1 according to the present invention shows that the addition of an additive such as phenol in a hydrocarbon solvent such as heptane improves the head-to-head dimer selectivity.

On the contrary, example 2 shows that the addition of phenol in a protic solvent such as isopropanol gives a head-to-head dimer selectivity that is less than the head-to-head selectivity of example 1.

The inventors have noted that the conversion can be increased by increasing the amount of phenol.

The inventors have also found that the selectivity for phenoxy-dimers decreases if the reaction time is increased.

## Claims

1. A process for the dimerization of conjugated diene compounds comprising contacting, in a solvent medium comprising hydrocarbons, said conjugated diene compounds with a catalyst in the presence of a phenol compound.

2. The process according to claim 1, wherein the conjugated diene compounds are terminal conjugated diene compounds

3. The process according to claim 2, wherein the terminal conjugated diene compounds have the following formula : 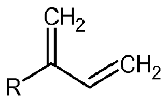 wherein R is a hydrocarbon radical having 1 to 15 carbon atom, optionally comprising one or more heteroatoms, such as nitrogen, oxygen or sulphur.

4. The process according to any one of claim 1 to 3, wherein the catalyst is a homogeneous catalyst.

5. The process according to any one of claim 1 to 3, wherein the catalyst is a heterogeneous catalyst.

6. The process according to any one of claims 1 to 5, wherein the solvent medium comprises at least 50% by weight of hydrocarbons, preferably at least 70% by weight, more preferably at least 90% by weight of hydrocarbons, still more preferably at least 99% by weight of hydrocarbons.

7. The process according to any one of claims 1 to 6, wherein the hydrocarbons comprised in the solvent are chosen from pentane, heptane, hexane, cyclohexane, toluene and xylene.

8. The process according to any one of claims 1 to 7, wherein the phenol compound is phenol.

9. The process according any one of claim 1 to 8, further comprising a hydrogenation step, whereby hydrogenated dimers are obtained.
